# EUROPEAN PATENT APPLICATION

(11) **EP 3 892 286 A1**
(43) Date of publication of application: **13.10.2021**
(21) Application number: 19893345.9
(22) Date of filing: 06.12.2019
(51) Int. Cl.: A61K 36/185, A61P 27/02, A23L 33/105

(54) **PHARMACEUTICAL COMPOSITION CONTAINING AUCUBA JAPONICA EXTRACT FOR PREVENTING OR TREATING MACULAR DEGENERATION**

(30) Priority: 07.12.2018 KR 20180157405
(71) Applicant: Industrial Cooperation Foundation Chonbuk National University, Jeonju-si, Jeollabuk-do 54896 (KR)
(72) Inventor: KIM, Jung Hyun, Jeonju-si, Jeollabuk-do 54987 (KR)
(74) Representative: Botti, Mario
(86) International application number: PCT/KR2019/017206
(87) International publication number: WO 2020/116996

(57) **Abstract**

The present disclosure relates to a pharmaceutical composition for preventing or treating macular degeneration, which contains *Aucuba japonica* extract.

## Description

### [Technical Field]

The present application claims priority of Korean Patent Application No. 10-2018-0157405 filed on December 7, 2018, the content of which is incorporated herein by reference in its entirety. The present disclosure relates to a pharmaceutical composition for preventing or treating macular degeneration, which contains *Aucuba japonica* extract.

### [Background Art]

The macula refers to the nerve tissue in the inner retina of the eye. Since cone cells are concentrated and the visual image of an object is focused at the center of the macula, it plays a very important role in vision. Vision refers to the ability of perceive the presence and shape of an object. The vision is the most sensitive when the image of the object is focused at the fovea centralis of the macula (central vision) and becomes weaker toward the periphery of the retina (peripheral vision). If the degradation of the macula occurs or its function declines due to aging, genetic factors, toxicity, inflammation, etc., vision is worsened or can be completely lost in severe cases. Macular degeneration refers to a disease caused by damage to the macula, which is important in vision. Macular degeneration is largely divided into dry and wet macular degeneration. In the dry macular degeneration, cellular debris called drusen accumulates on the retina, causing lesions such as thinning of retinal pigment epithelium. It is the common form of age-related macular degeneration and makes up about 90% of all cases. Although it usually does not cause severe vision loss, regular observation and prevention are important because it can develop into the wet form. The wet macular degeneration accounts for about 10% of all macular degeneration, and refers to a disease caused by choroidal neovascularization beneath the retina. The neovascularization affects central vision by causing exudation, bleeding, etc. at the macula, which is a very important part in the retina of the eye, leading to blindness in severe cases. Although various therapies have been developed for macular degeneration, there is no fundamental cure for suppressing the progress of symptoms, and prevention is more necessary than treatment. For example, Korean Patent Publication No. 10-2011-0021309 discloses a composition for treating, preventing or improving macular degeneration, which contains *Vaccinium uliginosum* extract or *Vaccinium uliginosum* fraction as an active ingredient, and Korean Patent Publication No. 10-2014-0045263 discloses a composition for preventing and treating vision deterioration and macular degeneration disease through retinal repair by ginsenoside Rg1.

*Aucuba japonica* is a broad-leaf shrub in the family *Garryaceae* growing around the southern sea and in Jeju Island in Korea. It is a garden tree growing in warm mountainous areas. It is the representative type of temperate forests in the southern part of Korea, especially in Ulleungdo area. The fruit of *Aucuba japonica* is beautiful and popular for enjoyment. The leaf and bark are used for medicine with the herbal names doyeopsanho, cheongakpan or cheongmok. *Aucuba japonica* has traditionally been used to treat neuralgia or inflammations such as arthritis and gout. The inventors of the present disclosure have researched on a composition for preventing and treating macular degeneration derived from natural products. In doing so, they have identified that *Aucuba japonica* hot water extract and ethanol extract inhibit the photo-oxidation of lipofuscin, which causes dry macular degeneration, and that particularly the ethanol extract effectively inhibits the photo-oxidation of lipofuscin than the hot water extract. Through animal experiments using *Aucuba japonica* extract, they have identified that the *Aucuba japonica* extract can inhibit the damage of retinal ganglion cells occurring in dry macular degeneration and can inhibit choroidal neovascularization occurring in wet macular degeneration, and have completed the present disclosure.

### [Disclosure]

### [Technical Problem]

The inventors of the present disclosure have researched on a composition for preventing and treating macular degeneration derived from natural products. In doing so, they have identified that *Aucuba japonica* hot water extract and ethanol extract inhibit the photo-oxidation of lipofuscin, which causes dry macular degeneration, and that particularly the ethanol extract effectively inhibits the photo-oxidation of lipofuscin than the hot water extract. Through animal experiments using *Aucuba japonica* extract, they have identified that the *Aucuba japonica* extract can inhibit the damage of retinal ganglion cells occurring in dry macular degeneration and can inhibit choroidal neovascularization occurring in wet macular degeneration, and have completed the present disclosure.

The present disclosure is directed to providing a pharmaceutical composition for preventing or treating macular degeneration, which contains *Aucuba japonica* extract.

The present disclosure is also directed to providing a functional health food composition for preventing or treating macular degeneration, which contains *Aucuba japonica* extract.

### [Technical Solution]

The present disclosure may provide a pharmaceutical composition for preventing or treating macular degeneration, which contains *Aucuba japonica* extract.

The macular degeneration may be age-related macular degeneration.

The *Aucuba japonica* extract may be one extracted from the leaf, stem or a mixture thereof of *Aucuba japonica.*

The *Aucuba japonica* extract may be extracted with water, a C₁₋₄ lower alcohol or a mixture solvent thereof.

The *Aucuba japonica* extract may be extracted with a 20-40% (w/w) aqueous ethanol solution.

The present disclosure may provide a functional health food composition for preventing or treating macular degeneration, which contains *Aucuba japonica* extract.

The macular degeneration may be age-related macular degeneration.

The *Aucuba japonica* extract may be one extracted from the leaf, stem or a mixture thereof of *Aucuba japonica.*

The *Aucuba japonica* extract may be extracted with water, a C₁₋₄ lower alcohol or a mixture solvent thereof.

The *Aucuba japonica* extract may be extracted with a 20-40% (w/w) aqueous ethanol solution.

The present disclosure may provide a method for treating macular degeneration, which includes a step of administering a pharmaceutically effective amount of *Aucuba japonica* extract to a subject in need thereof.

The present disclosure may provide a use of *Aucuba japonica* extract for use in a composition for treating macular degeneration.

### [Advantageous Effects]

*Aucuba japonica* hot water extract and ethanol extract of the present disclosure have the effect of inhibiting the photo-oxidation of lipofuscin, which causes dry macular degeneration. In particular, the ethanol extract has an effectively inhibiting the photo-oxidation of lipofuscin as compared to the hot water extract. Through animal experiments using the *Aucuba japonica* extract, it was identified that the *Aucuba japonica* extract can inhibit the damage of retinal ganglion cells occurring in dry macular degeneration and can inhibit choroidal neovascularization occurring in wet macular degeneration.

### [Brief Description of Drawings]

FIG. 1 shows the HPLC analysis result of *Aucuba japonica* ethanol extract and *Aucuba japonica* hot water extract of the present disclosure.
FIG. 2 shows a result of investigating the lipofuscin (A2E) photo-oxidation inhibiting effect of *Aucuba japonica* ethanol extract and *Aucuba japonica* hot water extract of the present disclosure.
FIG. 3 shows a histopathological result of orally administering AJE at a concentration of 100 or 250 mg/kg/day for a week to an MNU-induced optic nerve degeneration animal model and measuring the thickness of the outer nuclear layer depending on optic nerve cell degeneration.
FIG. 4 shows a result of orally administering AJE at a concentration of 100 or 250 mg/kg/day for a week to an MNU-induced dry macular degeneration animal model and measuring electroretinogram (ERG).
FIG. 5 shows a result of orally administering AJE at a concentration of 100 or 250 mg/kg/day for a week to a diode laser-induced wet macular degeneration animal model and measuring choroidal neovascularization (CNV).

### [Best Mode]

Hereinafter, the present disclosure is described in detail.

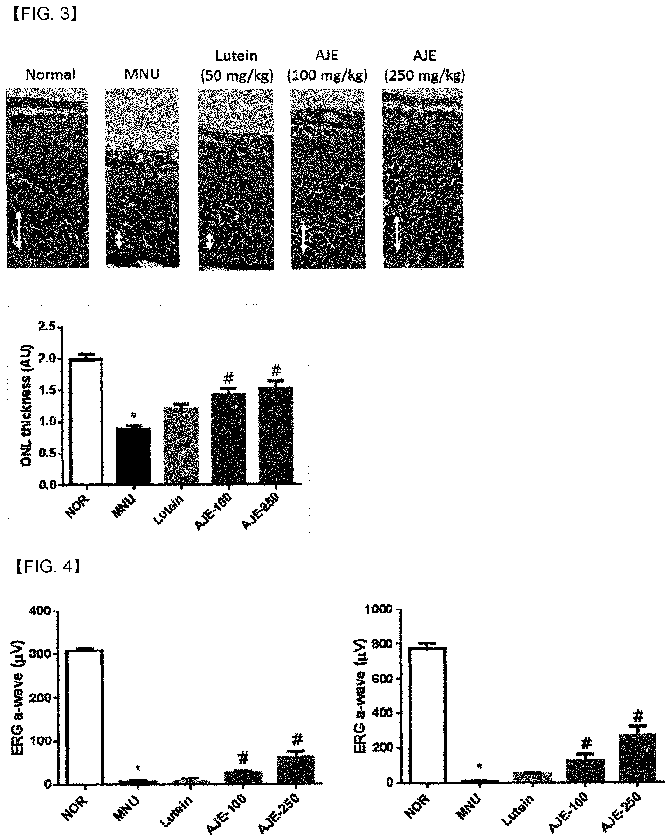

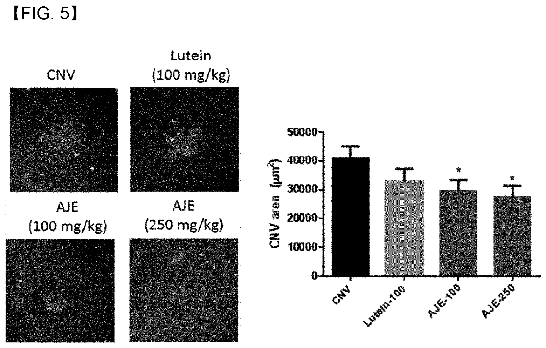

The inventors of the present disclosure have researched on a composition for preventing and treating macular degeneration derived from natural products. In doing so, they have identified that *Aucuba japonica* hot water extract and ethanol extract inhibit the photo-oxidation of lipofuscin (A2E), which causes dry macular degeneration, and that particularly the ethanol extract effectively inhibits the photo-oxidation of lipofuscin (A2E) than the hot water extract. Through animal experiments using *Aucuba japonica* extract, they have identified that the *Aucuba japonica* extract can inhibit the damage of retinal ganglion cells occurring in dry macular degeneration (FIG. 3 and FIG. 4) and can inhibit choroidal neovascularization occurring in wet macular degeneration (FIG. 5), and have completed the present disclosure.

The present disclosure is directed to providing a pharmaceutical composition for preventing or treating macular degeneration, which contains *Aucuba japonica* extract.

"*Aucuba japonica*" refers to a dicotyledonous evergreen fruit tree belonging to the family Garryaceae of the order Garryales. It is distributed mainly in the southern part of Korea, particularly in Ulleungdo area. It is also called doyeopsanho, cheongakpan or cheongmok.

According to the findings about the cause and progress of macular degeneration known thus far, the key risk factor of macular degeneration is the accumulation of A2E (pyridnium bisretinoid), which is not degraded in vivo, in the retinal pigment epithelium with aging. The macular degeneration may be age-related macular degeneration.

Macular degeneration is largely divided into dry and wet macular degeneration. In the dry macular degeneration, cellular debris called drusen accumulates on the retina, causing lesions such as thinning of retinal pigment epithelium. The wet macular degeneration refers to a disease caused by choroidal neovascularization beneath the retina. The inventors of the present disclosure have identified that *Aucuba japonica* extract treatment of an animal model in which dry macular degeneration was induced using N-methyl-N-nitrosourea (MNU, Sigma, USA) inhibits the thinning of the outer nuclear layer due to nerve cell degeneration in a concentration-dependent manner, improves inhibition of nerve conduction of a-wave and b-wave, and inhibits decrease in the activity of visual cells (FIG. 3 and FIG. 4). In addition, they disclosure have identified that *Aucuba japonica* extract treatment of an animal model in which wet macular degeneration was induced using diode laser inhibits choroidal neovascularization (CNV) in a concentration-dependent manner. Accordingly, it was confirmed that the *Aucuba japonica* extract is effective for both dry and wet macular degeneration (FIG. 5).

The extract may be an extract extracted from the whole aerial part of *Aucuba japonica,* a portion thereof or a material derived from thereof with a solvent. The portion may be the stem, leaf, flower, petal or seed of *Aucuba japonica*. Most specifically, it may be flower or stem. The whole *Aucuba japonica*, a portion thereof or a material derived from thereof used for the extraction may be one which has been pulverized, cut finely or dried adequately.

The *Aucuba japonica* extract may be extracted with water, a C₁₋₄ lower alcohol or a mixture solvent thereof, specifically with water or an aqueous ethanol solution, most specifically with an aqueous ethanol solution.

The ethanol may be a 20-40% (w/w) aqueous ethanol solution, specifically a 25-35% (w/w) ethanol aqueous solution, most specifically a 35% (w/w) ethanol aqueous solution.

The extraction may be performed by heated liquid extraction, pressurized liquid extraction (PLE), microwave-assisted extraction (MAE), subcritical extraction (SE) or a combination thereof. The subcritical extraction may be subcritical water extraction (SWE). The subcritical water extraction is also referred to as superheated water extraction or pressurized hot water extraction (PHWE). The heated liquid extraction may be reflux extraction.

The extraction may be performed at 4-70 °C, for example, at 4-50 °C, 4-40 °C, 4-30 °C, 10-70 °C, 15-70 °C, 20-70 °C, 4-50 °C, 10-50 °C, 4-40 °C, 4-30 °C, 10-40 °C, 10-35 °C or 10-30 °C. When the extraction is heated extraction, it may be performed at a temperature at which the solvent used for the extraction boils or at a temperature at which the desired ingredient is extracted from the plant. For example, it may be performed at 40 °C or higher, 50 °C or higher, 60 °C or higher, 70 °C or higher, 80 °C or higher, 90 °C or higher, 100 °C or higher, 120 °C or higher, 140 °C or higher, 160 °C or higher, or 200 °C or higher. As a specific example, it may be performed at 90-120 °C using water as a solvent. The time required for the extraction may vary depending on the selected temperature. The extraction time may be from 1 hour to 2 months, e.g., from 1 hour to 1 month, from 1 hour to 15 days, from 1 hour to 10 days, from 1 hour to 5 days, from 1 hour to 3 days, from 1 hour to 2 days, from 1 hour to 1 day, from 5 hours to 1 month, from 5 hours to 15 days, from 5 hours to 10 days, from 5 hours to 5 days, from 5 hours to 3 days, from 5 hours to 2 days, from 5 hours to 1 day, from 10 hours to 1 month, from 10 hours to 15 days, from 10 hours to 10 days, from 10 hours to 5 days, from 10 hours to 3 days, or from 10 hours to 2 days. The extraction may include mixing the whole aerial part of *Aucuba japonica,* a portion thereof, a seed or a material derived therefrom in the solvent and then waiting for a predetermined time. The waiting may include adequate stirring. The extraction may be repeated more than once, e.g., for 1-5 times.

The extraction may further include a process of separating a plant residue and an extracted solution by a known method such as filtration, etc. The extraction may include removing the solvent from the obtained extract by a known method such as concentration under reduced pressure. The extraction may also include drying the obtained extract by nitrogen drying or freeze-drying to prepare a dried extract. The extract may be resolvated using a suitable solvent after the drying, if necessary.

The composition may contain 0.001-80 wt%, e.g., 0.01-60 wt%, 0.01-40 wt%, 0.01-30 wt%, 0.01-20 wt%, 0.01-10 wt%, 0.01-5 wt%, 0.05-60 wt%, 0.05-40 wt%, 0.05-30 wt%, 0.05-20 wt%, 0.05-10 wt%, 0.05-5 wt%, 0.1-60 wt%, 0.1-40 wt%, 0.1-30 wt%, 0.1-20 wt%, 0.1-10 wt% or 0.1-5 wt%, of *Aucuba japonica* extract based on the total weight of the composition.

The composition may contain the extract in an effective amount or as an active ingredient depending on use. The effective amount may be selected adequately depending on a subject. It may be determined in consideration of the severity of a disease or condition, the age, body weight, health and sex of a subject, the subject's sensitivity to the extract, administration time, administration route, excretion rate, administration period, another composition used in combination with or simultaneously with the composition or other factors well known in the physiological or medical field.

The present disclosure may also provide a functional health food composition for preventing or treating macular degeneration, which contains *Aucuba japonica* extract.

When the composition is a functional health food composition, it may be prepared into a common functional health food formulation known in the art. The functional health food composition may be prepared into a general formulation such as a powder, a granule, a tablet, a pill, a capsule, a suspension, an emulsion, a syrup, an infusion, a liquid, an extract, etc., or may be prepared into any form of health food such as meat, sausage, bread, chocolate, candy, snack, confectionery, pizza, instant noodles or other noodles, gum, jelly, dairy products including ice cream, soups, beverages, tea, drinks, alcoholic beverages, vitamin complexes, etc. The health food may be prepared using a sitologically acceptable carrier or additive, and any carrier or additive known in the art as being usable for preparation of formulations may be used. The additive may include various nutrients, vitamins, electrolytes, flavorants, colorants, pectic acid and its salts, alginic acid and its salts, organic acids, protective colloidal thickeners, pH control agents, stabilizers, antiseptics, glycerin, alcohols, carbonating agents used in carbonated beverages, etc. In addition, a pulp for preparing natural fruit juice, fruit juice beverages and vegetable beverages may be included. These additive ingredients may be used independently or in combination, and the content of the additive may be 0.001-5 wt% or 0.01-3 wt% based on the total weight of the composition.

The content of the extract in the functional health food composition may be determined adequately depending on the purpose of use (for prevention or improvement). In general, it may be contained in an amount of 0.01-15 wt% of the total food weight and, when prepared as a beverage, may be contained in an amount of 0.02-10 g, specifically 0.3-1 g, per 100 mL. The beverage may further contain various flavorants, natural carbohydrates, etc. commonly used in beverages, in addition to the extract. The natural carbohydrate may include a common sugar such as a monosaccharide (e.g., glucose, fructose, etc.), a disaccharide (e.g., maltose, sucrose, etc.), a polysaccharide (e.g., dextrin, cyclodextrin, etc.) and a sugar alcohol such as xylitol, sorbitol, erythritol, etc. In addition, a natural flavorant (e.g., thaumatin, stevia extract, etc.) or a synthetic flavorant (e.g., saccharin, aspartame, etc.) may be contained as the flavorant. In general, the content of the natural carbohydrate may be about 1-20g, specifically about 5-12g, per 100 mL of the beverage.

When the composition is a pharmaceutical composition, it may contain a pharmaceutically acceptable diluent or carrier. The diluent may be lactose, corn starch, soybean oil, microcrystalline cellulose, mannitol, magnesium stearate, talc or a combination thereof. The carrier may be an excipient, a disintegrant, a binder, a lubricant or a combination thereof. The excipient may be microcrystalline cellulose, lactose, low-substituted hydroxyethyl cellulose or a combination thereof. The disintegrant may be calcium carboxymethyl cellulose, sodium starch glycolate, anhydrous dibasic calcium phosphate or a combination thereof. The binder may be polyvinylpyrrolidone, low-substituted hydroxypropyl cellulose, hydroxypropyl cellulose or a combination thereof. The lubricant may be magnesium stearate, silicon dioxide, talc or a combination thereof. The pharmaceutical composition may contain the extract in an effective amount or as an active ingredient. The effective amount may be selected adequately depending on a subject. It may be determined in consideration of the severity of a disease or condition, the age, body weight, health and sex of a subject, the subject's sensitivity to the extract, administration time, administration route, excretion rate, administration period, another composition used in combination with or simultaneously with the composition or other factors well known in the physiological or medical field.

The present disclosure may provide a method for treating macular degeneration, which includes a step of administering a pharmaceutically effective amount of *Aucuba japonica* extract to a subject in need thereof.

In the present disclosure, the "subject" refers to a subject in need of treatment of a disease. More specifically, it refers to any animal including human, monkey, cow, horse, sheep, pig, hen, turkey, quail, cat, dog, mouse, rat, rabbit or guinea pig. A disease may be prevented or treated effectively by administering the pharmaceutical composition of the present disclosure to the subject. The pharmaceutical composition of the present disclosure may be administered in combination with an existing therapeutic agent.

The present disclosure may provide a use of *Aucuba japonica* extract for use in preparing a composition for treating macular degeneration.

Hereinafter, the present disclosure will be described in more detail through examples. The examples are only for illustrating the present disclosure specifically, and it will be obvious those having ordinary knowledge in the art that the scope of the present disclosure is not limited by the examples.

### Example 1. Preparation of Aucuba japonica ethanol reflux extract

The leaf and stem of *Aucuba japonica* were harvested in the forest land of Jisepo-ri, Ilun-myeon, Geoje-si, Gyeongsangnam-do of Korea in August 2018. The samples are stored in the sample storage room of Jeonbuk University. After reflux extracting 50 g of *Aucuba japonica* (mixture of leaf and stem) once at 70-100 °C for 3 hours by adding to 1500 mL of 30% (w/w) ethanol, a 30% ethanol reflux extract was obtained by concentrating under reduced pressure and freeze-drying the same.

### Example 2. Preparation of Aucuba japonica hot water extract

The leaf and stem of *Aucuba japonica* were harvested in the forest land of Jisepo-ri, Ilun-myeon, Geoje-si, Gyeongsangnam-do of Korea in August 2018. The samples are stored in the sample storage room of Jeonbuk University. After adding 900 mL of distilled water to 50 g of *Aucuba japonica* (mixture of leaf and stem), an *Aucuba japonica* hot water extract was prepared by extracting at 100 °C for 3 hours, concentrating under reduced pressure and then drying the same.

### Example 3. Analysis of aucubin content through HPLC analysis of Aucuba japonica extract

### 3-1. Reagents and instruments

Standard aucubin (purity: 99.5%) was purchased from Biopurity Phytochemicals Ltd. (Chengdu, China). HPLC-grade acetonitrile and water (J.T. Backer, USA) were used as mobile phase solvents for HPLC analysis. The Agilent's 1200 analytical HPLC system used for HPLC analysis consisted of a binary pump (G1312A), a vacuum degasser (G1322A), a thermostatted column compartment (G1316A), a multiple wavelength detector (1365B, MWD) and an autosampler (G1329A). The ChemStation software was used for instrument operation and analysis result processing.

### 3-2. Preparation of standard solution and construction of calibration curve

Standard aucubin was dissolved in MeOH to a concentration of 2 mg/mL and then diluted serially to prepare standard solutions for constructing a calibration curve with concentrations of 2000, 1000, 500 and 100 µg/mL. After injecting each standard solution into a column, a chromatogram was obtained by conducting HPLC analysis. Then, a calibration curve for quantitative analysis may be obtained by plotting the peak.

### 3-3. Preparation of test solution

After adding 100 mg of *Aucuba japonica* extract to a 10-mL volumetric flask, 50% MeOH was added up to the calibration mark. After conducting ultrasonic extraction for 5 minutes, 1 mL of the prepared test solution was filtered through a 0.45-µm Whatman PVDF syringe filter and used for HPLC analysis.

### 3-4. HPLC analysis

Zorbax Eclipse Plus C18 (4.6 x 250 mm/5.0 µm, Agilent) was used for HPLC analysis. Column temperature was set to 35 °C, and the UV detector wavelength was set to 210 nm. 3 µL of the sample was injected using an autosampler. A mixture solution of water (A) and acetonitrile (B) was flown at a rate of 1.0 mL/min as a mobile phase. The concentration gradient of the mobile phase is shown in Table 1.

**[Table 1] Concentration gradient of HPLC analysis**

| Time (min) | Solvent A (%)^{a} | Solvent B (%)^{b} |
|---|---|---|
| 0 | 97 | 3 |
| 8 | 97 | 3 |
| 30 | 75 | 25 |

As a result of HPLC-DAD analysis, standard aucubin was detected around 11.33 minutes in the chromatogram. When a calibration curve was constructed in the aucubin concentration range of 100-2000 µg/mL, a good linearity with a correlation coefficient (R²) of 0.999 or higher was achieved. The content of aucubin in the *Aucuba japonica* leaf and stem extracts was analyzed using the established analysis method. After calculating the peak area of aucubin in the chromatogram of the *Aucuba japonica* extract, the aucubin content was calculated by comparing with the calibration curve. The aucubin content analysis result for each extract is shown in Table 2.

**[Table 2]**

| Extract | Aucubin content (mean ± SD, n = 3) | |
|---|---|---|
| | mg/g | % |
| Hot water extract | 43.22 ± 1.21 | 4.32 |
| 30% ethanol extract | 64.41 ± 1.15 | 6.44 |

### Example 4. Analysis of lipofuscin (A2E) photo-oxidation inhibiting effect of Aucuba japonica extract

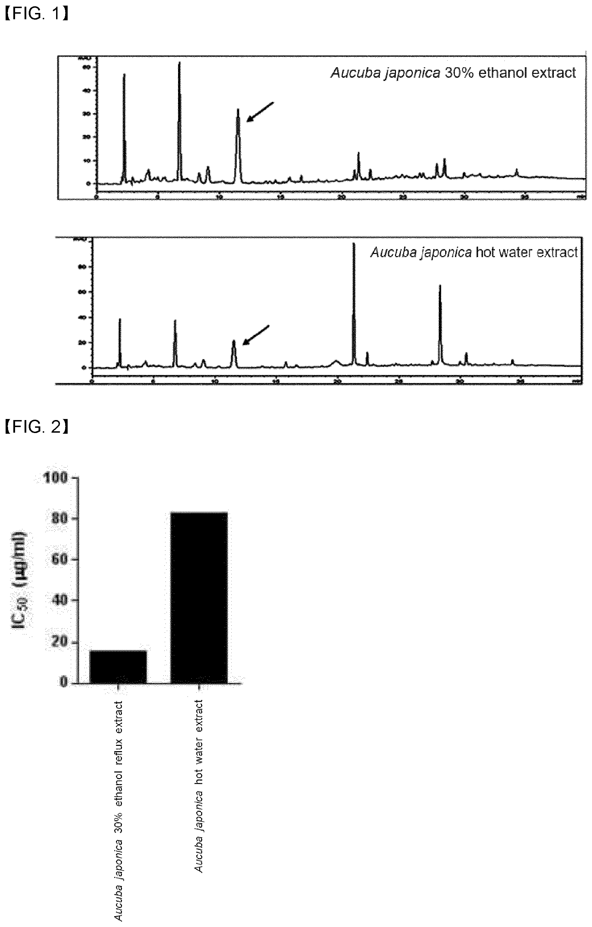

After dissolving A2E (Gene And Cell Technologies, USA) in PBS containing 0.5% DMSO to 100 µM and then adding to a 96-well microplate with 180 µL per well, 20 µL of a vehicle (control) or the *Aucuba japonica* extract of Example 1 or Example 2 was added to each well. The mixture was exposed to blue light (430 nm, intensity: 2.0-2.5 mW/cm²) for 10 minutes. The degree of photo-oxidation of A2E before and after UV A irradiation was evaluated by measuring absorbance at 440 nm using a microplate leader. Lipofuscin (A2E) is a substance which causes macular degeneration. It is accumulated in the cells of the retinal pigment epithelium and exhibits cytotoxicity when photo-oxidized by blue light, etc. As seen from FIG. 2, the *Aucuba japonica* 30% ethanol extract showed an inhibitory concentration 50% (IC₅₀) of inhibiting the photo-oxidation of lipofuscin by 50% of 15.2 µg/mL, and the hot water extract had an IC₅₀ of 82.6 µg/mL. The effect of the 30% ethanol extract was about 5 times superior as compared to the hot water extract. Accordingly, all the subsequent tests using animal models were performed using the *Aucuba japonica* 30% ethanol extract (hereinafter, referred to as "AJE").

### Example 5. Investigation of effect of Aucuba japonica extract in dry macular degeneration animal model (N-methyl-N-nitrosourea (MNU)-induced photoreceptor cell degeneration mouse)

### 5-1. Histopathological evaluation

6-week-old male C57BL/6 mice were purchased from Orient (Seongnam, Korea) and used after accustomation for a week. In order to induce damage and degeneration of photoreceptor cells from among the morphological changes of retinal tissue occurring in dry macular degeneration, N-methyl-N-nitrosourea (MNU, Sigma, USA) was prepared in a 0.05% acetic acid solution to a concentration of 1%. The 1% MNU solution was intraperitoneally administered to the 7-week-old male C57BL/6 mice with 60 mg/kg per each. Only 0.05% acetic acid of the same volume was intraperitoneally administered to a normal group. The *Aucuba japonica* 30% ethanol extract (AJE) was dissolved in sterilized triply distilled water to a concentration of 100 or 250 mg/kg/day and administered orally from the day of MNU administration once a day for 7 days. As a control drug, lutein was orally administered at a concentration of 50 mg/kg/day for the same period. After the administration of the test substance, the eye was extracted by autopsy, fixed in 10% neutralizing formalin for a day, and then embedded in paraffin to prepare slide sections. The slide sections were stained with hematoxylin and eosin (H&E) and then observed under an optical microscope. The damage and degeneration of photoreceptor cells were evaluated by measuring the change in the thickness of the outer nuclear layer of the retinal tissue. It was confirmed that the oral administration of the *Aucuba japonica* 30% ethanol extract (AJE) to the MNU-induced optic nerve degeneration animal model at a concentration of 100 or 250 mg/kg/day for a week inhibited the decrease of the thickness of the outer nuclear layer due to optic nerve cell degeneration in a concentration-dependent manner (FIG. 3). In particular, it was confirmed that the *Aucuba japonica* 30% ethanol extract (AJE) showed better effect than lutein which is widely used for prevention of dry macular degeneration.

### 5-2. Measurement of electroretinogram (ERG)

An electroretinogram (ERG) represents retinal optic nerve conduction in response to light, and is used as an indicator of retinal damage or disease. The inventors of the present disclosure have measured an electroretinogram. One day before autopsy, the mouse was subjected to scotopic adaptation for at least 16 hours. For ERG measurement, the mouse was anesthetized with isoflurane (Forane, JW Pharmaceutical) and the pupil was dilated with 0.5% tropicamide. While maintaining body temperature under general anesthesia, an appropriate amount of 1% methylcellulose was applied onto the eye. ERG measurement was made using Retiport (Roland consult, Germany) by contacting a gold wire loop recording electrode with the cornea, contacting a reference electrode with the cheek, and contacting a ground electrode with the tail. Stimulation was generated with white LED light, and a- and b-waves were obtained from scotopic flash response. In general, an ERG consists of a- and b-waves. The a-wave is generated by photoreceptors, and the b-wave is generated as the bipolar cells in the inner nuclear layer are depolarized by optical stimulation. As a result of measuring the electroretinography (ERG) for the animal model, it was confirmed that the administration of the *Aucuba japonica* 30% ethanol extract (AJE) improves the inhibition of nerve conduction of a- and b-waves and inhibits the decrease in the activity of visual cells.

### Example 6. Investigation of effect of Aucuba japonica extract (AJE) in wet macular degeneration animal model (laser-induced choroidal neovascularization (CNV) rat

7-week-old male Brown Norway (BN) rats (SLC Japan, Tokyo, Japan) were accustomed for a week and then anesthetized by intraperitoneally injecting pentobarbital sodium (Hanlim Pharm, 25 mg/kg). Then, after dilating the pupil by dropping 1% tropicamide, six photocoagulation spots were created around the optic nerve head using a diode laser (wavelength: 532 nm, diameter: 100 µm, power: 150 mW, duration: 0.1 sec). The destruction of Bruch's membrane was verified by the formation of characteristic bubbles. After the photocoagulation treatment, the male rats were randomly divided into 5 groups of 10 per each, and were orally administered with the corresponding test substance for 10 days. The test substance was dissolved in sterilized triply distilled water to a concentration of 100 or 250 mg/kg/day and then was orally administered from the day of the photocoagulation treatment once a day for 10 days. As a control drug, lutein was orally administered at a concentration of 100 mg/kg/day for the same period. 10 days after the drug administration, the animal was sacrificed and the eye was extracted. After excising the eye near the cornea and the sclera under a microscope, the retina detached and the conjunctival tissue including the subretinal region was separated. The separated tissue was fixed in 4% paraformaldehyde for 1 hour, washed with PBS, stirred with PBS containing 5% Triton X-100 and 1% BSA for 3 hours, washed again, and then incubated overnight at 4 °C with the endothelial cell marker isolectin B4 (Sigma) dissolved in PBS to 1 mg/mL and diluted to 1:50. After washing with PBS containing 0.05% Tween 20 for 2 hours and then incubating with streptavidin TRITC diluted to 1:500 at 37 °C for 4 hours, followed by washing with PBS for 30 minutes, the size of subretinal neovascularization was analyzed with the Image J software (NIH, USA) after observation under a fluorescence microscope (BX51, Olympus, Japan). Wet macular degeneration is characterized by formation of immature new blood vessels and edema in the subretinal region. As a result of inducing choroidal neovascularization (CNV) by disrupting Bruch's membrane using a laser and then orally administering AJE at a concentration of 100 or 250 mg/kg/day for 10 days, it was confirmed that CNV was inhibited in a concentration-dependent manner. In particular, it was confirmed that the AJE exhibits better effect than lutein which is widely used for prevention of dry macular degeneration.

## Claims

1. A pharmaceutical composition for preventing or treating macular degeneration, comprising *Aucuba japonica* extract.

2. The pharmaceutical composition for preventing or treating macular degeneration according to claim 1, wherein the macular degeneration is age-related macular degeneration.

3. The pharmaceutical composition for preventing or treating macular degeneration according to claim 1, wherein the *Aucuba japonica* extract is one extracted from the leaf, stem or a mixture thereof of *Aucuba japonica.*

4. The pharmaceutical composition for preventing or treating macular degeneration according to claim 1, wherein the *Aucuba japonica* extract is extracted with water, a C₁₋₄ lower alcohol or a mixture solvent thereof.

5. The pharmaceutical composition for preventing or treating macular degeneration according to claim 1, wherein the *Aucuba japonica* extract is extracted with a 20-40% (w/w) aqueous ethanol solution.

6. A functional health food composition for preventing or treating macular degeneration, comprising *Aucuba japonica* extract.

7. The functional health food composition for preventing or treating macular degeneration according to claim 6, wherein the macular degeneration is age-related macular degeneration.

8. The functional health food composition for preventing or treating macular degeneration according to claim 6, wherein the *Aucuba japonica* extract is one extracted from the leaf, stem or a mixture thereof of *Aucuba japonica.*

9. The functional health food composition for preventing or treating macular degeneration according to claim 6, wherein the *Aucuba japonica* extract is extracted with water, a C₁₋₄ lower alcohol or a mixture solvent thereof.

10. The functional health food composition for preventing or treating macular degeneration according to claim 6, wherein the *Aucuba japonica* extract is extracted with a 20-40% (w/w) aqueous ethanol solution.

11. A method for preventing and treating macular degeneration, comprising a step of administering a pharmaceutically effective amount of *Aucuba japonica* extract to a subject in need thereof.

12. A use of *Aucuba japonica* extract for use in a composition for preventing and treating macular degeneration.
